# EUROPEAN PATENT SPECIFICATION

(11) **EP 3 978 012 B1**
(45) Date of publication and mention of the grant of the patent: **16.08.2023**
(21) Application number: 21806931.8
(22) Date of filing: 30.03.2021
(51) Int. Cl.: A61K 38/44, A61P 1/16

(54) **FLAVIN-CONTAINING MONOOXYGENASE 2 FOR TREATMENT OF NON-ALCOHOLIC FATTY LIVER DISEASE**
FLAVINHALTIGER MONOOXYGENASE 2 (FMO2) ZUR BEHANDLUNG VON NICHT-ALKOHOLISCHER FETTLEBERERKRANKUNG (NAFLD)
MONOOXYGÉNASE 2 CONTENANT DE LA FLAVINE POUR LE TRAITEMENT D'UNE STÉATOSE HÉPATIQUE NON ALCOOLIQUE

(30) Priority: 17.08.2020 CN 202010825464
(43) Date of publication of application: 06.04.2022
(73) Proprietor: Zhejiang University, Hangzhou, Zhejiang 310058 (CN)
(72) Inventor: HU, Xinyang, Hangzhou Zhejiang 310058 (CN); WANG, Jianan, Hangzhou Zhejiang 310058 (CN); KE, Changle, Hangzhou Zhejiang 310058 (CN)
(74) Representative: Elzaburu S.L.P.
(86) International application number: PCT/CN2021/083861
(87) International publication number: WO 2022/037070

(56) References cited:
- WO-A1-2014/052960
- CN-A- 109 200 276
- CN-A- 110 115 760
- CN-A- 111 973 736
- SHIH DIANA M. ET AL: "Flavin containing monooxygenase 3 exerts broad effects on glucose and lipid metabolism and atherosclerosis", JOURNAL OF LIPID RESEARCH, vol. 56, no. 1, 1 January 2015 (2015-01-01), pages 22-37, XP055948663, US ISSN: 0022-2275, DOI: 10.1194/jlr.M051680
- ANGULO PAUL: "NONALCOHOLIC FATTY LIVER DISEASE", NEW ENGLAND JOURNAL OF MEDICINE, THE - NEJM, MASSACHUSETTS MEDICAL SOCIETY, US, vol. 346, no. 16, 18 April 2002 (2002-04-18), pages 1221-1231, XP009072729, ISSN: 1533-4406, DOI: 10.1056/NEJMRA011775
- SHI CHUNXIA ET AL: "Changes of flavin-containing monooxygenases and trimethylamine-N-oxide may be involved in the promotion of non-alcoholic fatty liver disease by intestinal microbiota metabolite trimethylamine", BIOCHEMICAL AND BIOPHYSICAL RESEARCH COMMUNICATIONS, ELSEVIER, AMSTERDAM NL, vol. 594, 16 January 2022 (2022-01-16), pages 1-7, XP086947049, ISSN: 0006-291X, DOI: 10.1016/J.BBRC.2022.01.060 [retrieved on 2022-01-16]
- Gornicka Agnieszka, Morris-Stiff Gareth, Thapaliya Samjhana, Papouchado Bettina G., Berk Michael, Feldstein Ariel E.: "Transcriptional Profile of Genes Involved in Oxidative Stress and Antioxidant Defense in a Dietary Murine Model of Steatohepatitis", ANTIOXIDANTS AND REDOX SIGNALING, MARY ANN LIEBERT, LARCHMONT, NY, US, vol. 15, no. 2, 15 July 2011 (2011-07-15), pages 437-445, XP055902173, US ISSN: 1523-0864, DOI: 10.1089/ars.2010.3815
- Lebeau Paul F., Byun Jae Hyun, Platko Khrystyna, Al-Hashimi Ali A., Lhoták ?árka, Macdonald Melissa E., Mejia-Benitez Aurora, Prat: "Pcsk9 knockout exacerbates diet-induced non-alcoholic steatohepatitis, fibrosis and liver injury in mice", JHEP Reports, vol. 1, no. 6, 1 December 2019 (2019-12-01), pages 418-429, XP055902171, ISSN: 2589-5559, DOI: 10.1016/j.jhepr.2019.10.009

## Description

### TECHNICAL FIELD

The present invention relates to the field of biomedicine, and in particular relates to an application of flavin-containing monooxygenase 2 (FMO2) for use in the treatment of non-alcoholic fatty liver disease (NAFLD) and to a composition comprising FMO2 for use in the treatment of NAFLD.

### BACKGROUND ART

Non-alcoholic fatty liver disease (NAFLD) refers to a clinical syndrome having a main pathological feature of excessive deposition of lipids (mainly triglycerides) in liver parenchymal cells not caused by drinking or other clear reasons. NAFLD is a progressive liver disease, divided into four stages from mild to severe: non-alcoholic fatty degeneration of liver, nonalcoholic steatohepatitis (NASH), liver cirrhosis, and hepatocellular carcinoma. According to related surveys, the prevalence of NAFLD worldwide is 25%, and about 30% of the population in China suffers from the disease. In particular, with the progress of society and the continuous improvement of living standards, intake of a lot of high-fat foods and imbalance of the dietary structure have caused the incidence of NAFLD to increase year by year and a rapid trend of youth of the affected population.

NAFLD is not an independent disease, and its pathogenesis is complicated. The classic "two hits" theory believes that a large amount of fat accumulated in the liver parenchymal cells caused by various reasons (especially an increase in the insulin concentration) is the first hit, and lipid peroxidation and oxidative stress are the second hit. In recent years, with the deepening of research, a "multiple hits" theory has gradually been accepted by people, and it believes that the pathogenesis of NAFLD is not limited to the two hits mentioned above, and shall also include other factors such as inflammatory cytokines, host-microbe interactions, genetic factors, and dietary factors. At present, there are no really effective therapeutic drugs for NAFLD in clinical practice, and the treatment mainly focuses on removal of the inducement and adjustment of the diet. Therefore, it is very necessary to develop high-efficiency, low-side-effect drugs for the treatment of NAFLD.

Flavin-containing monooxygenase (FMO) belongs to the family of flavoproteinases, and is a group of microsomal enzymes relying on flavin adenine dinucleotide (FAD), reduced nicotinamide adenine dinucleotide phosphate (NADPH) and molecular oxygen. FMO is generally found in eukaryotes. Due to the characteristics of regional selection, chiral catalysis, good stability, and the like, FMO has received extensive attention in the fields of xenobiotic metabolism, pharmacokinetics, biocatalytic synthesis, and the like. FMO is the second important drug metabolic enzyme after the cytochrome P450 (CYPs) enzyme, is a very important enzyme in detoxification and elimination of drugs and chemical substances, and can catalyze oxidation of compounds and drugs containing nitrogen, sulfur, phosphorus, selenium and other nucleophilic heteroatoms. FMO exists in many subtypes of mammals and humans, and each has its substrate specificity and tissue limitations. Although all isomers of human FMO have about 60% sequence similarity, their structural and functional similarities are very low. Studies have reported that the lack or insufficiency of FMO3 can lead to trimethylamine metabolism disorders, which is the cause of "fish smell syndrome", that is, trimethylaminuria.

Furthermore, FMO2 is a member of the flavin-containing monooxygenase family, and is an enzyme with NADP+ and FAD as prosthetic groups and having biological metabolism. After the protein was discovered in 1992, there was no follow-up study of its function for a long time. Until 2015, studies have found that FMO2 is related to the lifespan and activity of nematodes. So far, the research on FMO2 has mainly focused on gene polymorphism, and there are few studies on the function of FMO2. Compared with CYPs, FMOs have long been ignored. However, with human research on the characteristics of FMO family members and the consequences of rare mutations and common polymorphic mutations of FMO, people have become more and more aware of the pharmacological and toxicological significance of these enzymes. As an important enzyme in the liver, FMO has huge research value.

### SUMMARY OF THE INVENTION

In order to solve the above technical problems, the present invention provides an application of flavin-containing monooxygenase 2 (FMO2) for use in the treatment of non-alcoholic fatty liver disease (NAFLD). The present invention finds for the first time that FMO2 has an effect of preventing or treating NAFLD.

The specific technical solutions of the present invention are as follows:
In the first aspect, the present invention provides an application of FMO2 for use in the treatment of NAFLD.

The present invention finds that FMO2 can achieve the effect of treating fatty liver by inhibiting de novo lipid synthesis mediated by SREBP1/ACC/FASN pathways in the liver.

Preferably, the FMO2 has a nucleotide sequence as shown in SEQ ID NO. 1 (mice) or SEQ ID NO. 3 (human), and an amino acid sequence as shown in SEQ ID NO. 2 (mice) or SEQ ID NO. 4 (human).Preferably, the FMO2 is administered as an oral preparation or an injection preparation.

Preferably, the dosage form of the oral preparation is a capsule, a tablet, a solution or powder; and the injection preparation is an injection or powder.

In the second aspect, the present invention provides composition comprising FMO2 for use in the treatment of NAFLD, wherein said composition further comprises a pharmaceutically acceptable carrier, excipient or solvent.

Compared with the prior art, the present invention has the beneficial effect that the present invention finds that FMO2 has an effect of preventing or treating NAFLD.

### BRIEF DESCRIPTION OF THE DRAWINGS

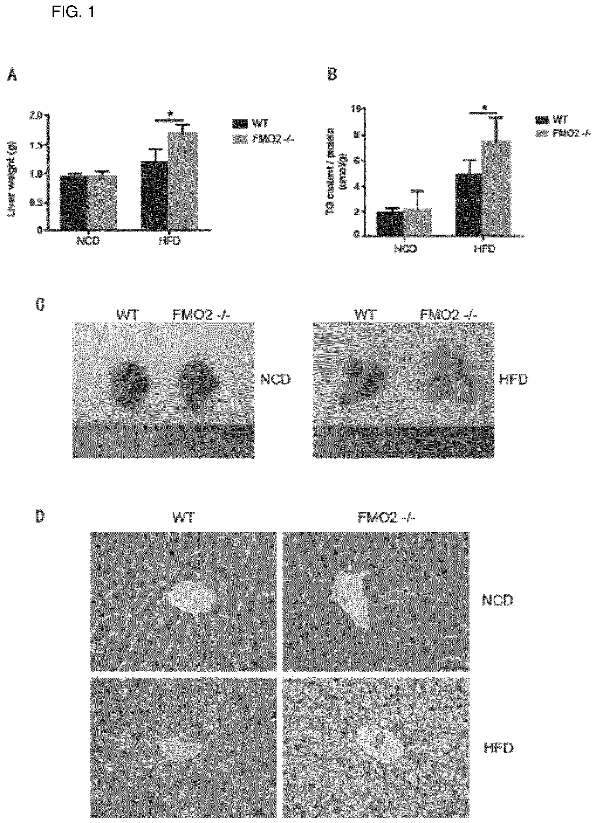

FIG. 1 is a graph showing the results of in vivo experiments on C57 mice fed with a normal control diet or a high-fat diet for 12 weeks. A is the statistical result of liver weight of wild-type mice and FMO2^{-/-} mice, *P<0.05 WT Versus FMO2^{-/-}; B is the statistical result of liver triglyceride content of the wild-type mice and FMO2^{-/-} mice, *P<0.05 WT Versus FMO2^{-/-}; C is the comparison result of general appearance of liver of the wild-type mice and FMO2^{-/-} mice; and D is the comparison result of HE staining of liver paraffin sections of the wild-type mice and FMO2^{-/-} mice. WT represents the wild-type mouse group and FMO2^{-/-} represents the FMO2 knockout transgenic mouse group. NCD represents the normal control diet group, and HFD represents the high-fat diet group.

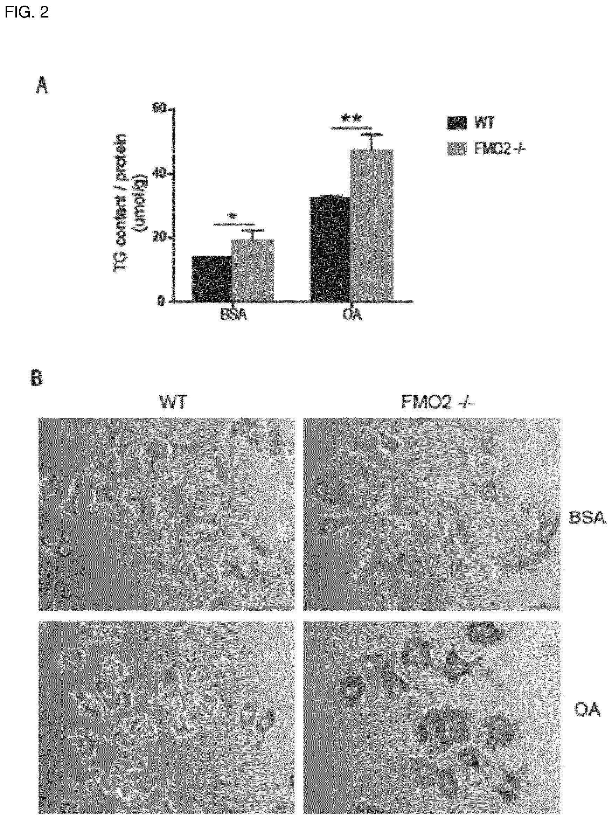

FIG. 2 is the results of in vitro experiments on primary hepatocytes of C57 mice. A is the statistical result of the triglyceride content of wild-type primary hepatocytes and FMO2^{-/-} primary hepatocytes, *P<0.05, **P<0.01 WT Versus FMO2^{-/-}; and B is the result of oil red staining of the wild-type primary hepatocytes and FMO2^{-/-} primary hepatocytes. BSA represents a normal control group, and OA represents a fatty liver in vitro model group.

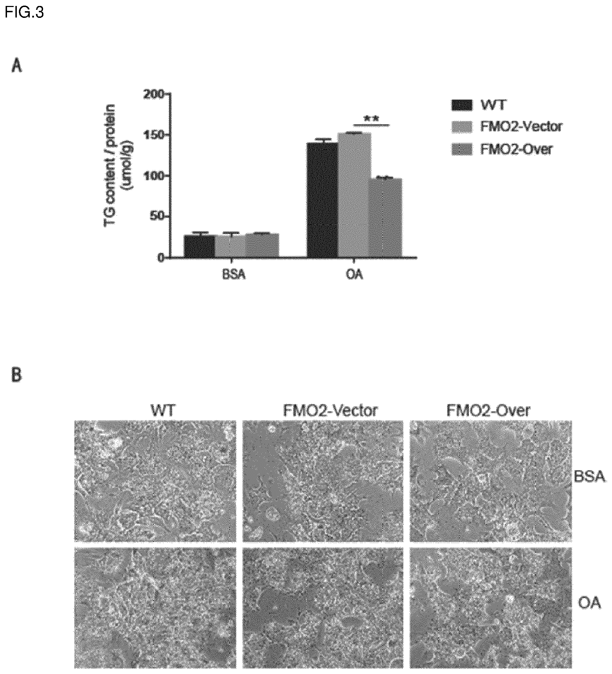

FIG. 3 is the results of in vitro experiments on primary hepatocytes of C57 mice. A is the statistical result of the triglyceride content of the wild-type primary hepatocyte group, the primary hepatocyte group transfected with the FMO2 negative control virus, and the primary hepatocyte group transfected with the FMO2 overexpression virus, **P<0.01 WT Versus FMO2^{-/-}; and B is the oil red staining results of the wild-type primary hepatocyte group, the primary hepatocyte group transfected with the FMO2 negative control virus, and the primary hepatocyte group transfected with the FMO2 overexpression virus. WT represents the wild-type mouse primary hepatocyte group, FMO2-Vector represents the primary hepatocyte group transfected with the FMO2 negative control virus, and FMO2-Over represents the primary hepatocyte group transfected with the FMO2 overexpression virus. BSA represents a normal control group, and OA represents a fatty liver in vitro model group.

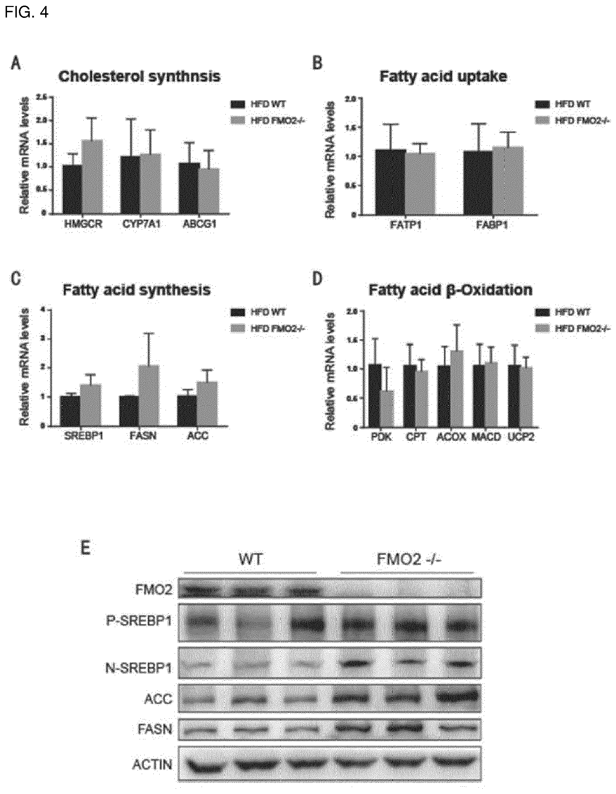

FIG. 4 is the experimental results of the in vivo mechanism study on C57 mice. A is the expression of cholesterol synthesis-related genes in the livers of the wild-type mice and FMO2^{-/-} mice fed with the high-fat diet for 12 weeks; B is the expression of fatty acid uptake-related genes in the livers of the wild-type mice and FMO2^{-/-} mice fed with the high-fat diet for 12 weeks; C is the expression of fatty acid and triglyceride synthesis-related genes in the livers of the wild-type mice and FMO2^{-/-} mice fed with the high-fat diet for 12 weeks; D is the expression of fatty acid and triglyceride oxidative metabolism-related genes in the livers of the wild-type mice and FMO2^{-/-} mice fed with the high-fat diet for 12 weeks; and E is the Western blot results of key proteins of the lipid synthesis pathways in the livers of the wild-type mice and FMO2^{-/-} mice fed with the high-fat diet for 12 weeks. WT represents the wild-type mouse group and FMO2^{-/-} represents the FMO2 knockout transgenic mouse group.

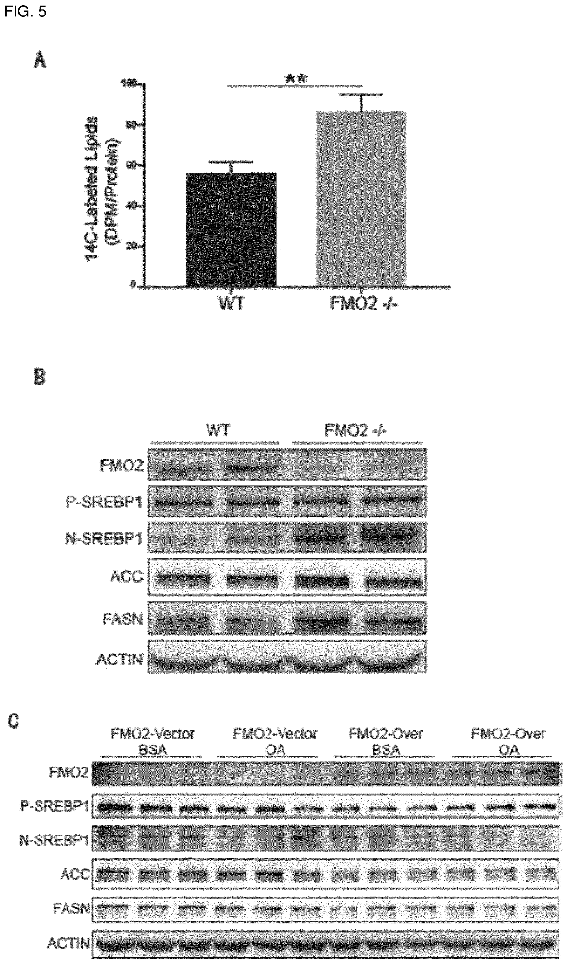

FIG. 5 is a graph showing the experimental results of in vitro mechanism study on mouse primary hepatocytes. A is the results of lipid synthesis experiments, recording the lipid de novo synthesis ability of wild-type primary hepatocytes and FMO2^{-/-} primary hepatocytes; B is the Western blot results of key proteins of the lipid synthesis pathways in the wild-type primary hepatocytes and FMO2^{-/-} primary hepatocytes; and C is the Western blot results of key proteins of the lipid synthesis pathway in the FMO2 negative control virus-transfected primary hepatocyte group and the FMO2 overexpression virus-transfected primary hepatocyte group.

### DETAILED DESCRIPTION OF THE INVENTION

The present invention will be further described below in conjunction with embodiments.

A flavin-containing monooxygenase 2 (FMO2) for use in the treatment of non-alcoholic fatty liver disease (NAFLD).

Preferably, the FMO2 has a nucleotide sequence as shown in SEQ ID NO. 1 (mice) or SEQ ID NO. 3 (human), and an amino acid sequence as shown in SEQ ID NO. 2 (mice) or SEQ ID NO. 4 (human).

Preferably, the FMO2 is administered as an oral preparation or an injection preparation. Further, the dosage form of the oral preparation is a capsule, a tablet, a solution or powder; and the injection preparation is an injection or powder.

A composition comprising FMO2 for use in the treatment NAFLD, wherein said composition further comprises a a pharmaceutically acceptable carrier, excipient or solvent.

### Example 1

Preparation of flavin-containing monooxygenase 2 (FMO2) overexpression virus:
In the embodiments of the present invention, FMO2 overexpression lentivirus (Ubi-FMO2-3FLAG-CBh-gcGFP-IRES-puromycin), and negative control lentivirus (Ubi-MCS-3FLAG-CBh-gcGFP-IRES-puromycin) were all purchased from Shanghai Genechem Co., LTD.; and a mouse FMO2 nucleotide sequence was as shown in SEQ ID NO. 1.

### Example 2

### 1. Establishment of a mouse model of NAFLD

In the present invention, 8 week-old healthy C57BL/6J mice were divided into four groups, that is, a wild-type mouse normal control diet group, a wild-type mouse high-fat diet group, an FMO2 knockout mouse normal control diet group, and an FMO2 knockout mouse high-fat diet group. 5 mice in each group were fed for 12 weeks.

### 2. Extraction, fixation, sectioning, and staining of tissue

(1) Extraction: After the mice were anesthetized with 1 % pentobarbital sodium, the abdominal cavity was quickly opened, and the liver was quickly separated after blood was collected from the heart. After weighing, an appropriate amount of liver tissue was cut and put into prepared tissue lysis buffer, triglyceride lysis buffer and 4% paraformaldehyde fixative.
(2) Fixation and sectioning: After being fixed with 4% paraformaldehyde, the liver was divided into two parts for paraffin embedding and OCT embedding respectively. The paraffin-embedded tissue was sectioned serially (with a thickness of 3 µm). After being picked out, the sections were placed in a 60°C oven, baked for 30 min, and placed in a clean section box for later use. During OCT embedding, the tissue was slowly immersed in liquid nitrogen to prevent the tissue block from freezing and cracking. 6 µm thick frozen sections were attached to a cationic glass slide, and dried and stored in a -80°C refrigerator for later use.
(3) HE staining: After being fixed with 4% paraformaldehyde for 10 min, the liver paraffin sections were washed with PBS for 5 min 3 times, subjected to nucleus staining with hematoxylin for 40 s, subjected to 1% hydrochloric acid (1 mL of concentrated hydrochloric acid with 99 mL of absolute ethanol added) alcohol differentiation and immersed 3 times, washed with water for 5 min 3 times, stained with eosin for 1 min, washed with water for 5 min 3 times, dehydrated with gradient alcohol (75%, 2 s; 85%, 2 s; 90%, 2 min; 95%, 2 min; 100%, 5 min/2 times), washed with xylene for 5 min 2 times, and mounted with resin.

### 3. Liver triglyceride detection

About 50 mg of liver tissue was taken, 1 ml of triglyceride lysis buffer was added, and the liver tissue was homogenized 30 times in a glass homogenizer and lysed for 10 min at room temperature. 100 µl of the homogenate was taken for BCA protein measurement, and the remaining homogenate was placed in a 70°C water bath for 10 min to inactivate lipase. The homogenate was centrifuged at 2000 rpm for 5 min at room temperature, the supernatant was taken for triglyceride quantification, and finally the quantity was converted to TG/protein in µmol/g.

### 4. RNA extraction, reverse transcription and real-time fluorescent quantitative PCR

1 ml of Trizol (Invitrogen) was added to 20 mg of tissue. After the tissue was homogenized, 200 µl of trichloromethane was added, shaken vigorously for mixing, and centrifuged at 12000 rpm for 15 min. The supernatant was transferred to the same volume of isopropanol, mixed by inversion, and centrifuged at 12000 rpm to obtain a precipitate. The precipitate was washed twice with 70% ethanol, and 50-100 µl of DEPC water was added. After the RNA concentration was measured, 1500 ng of total RNA was taken for reverse transcription. The reverse transcription uses the PrimeScript^{™} RT Master Mix reverse transcription kit from Takara. Fluorescent quantitative PCR uses the TB Green^{®} Premix Ex Taq^{™} II kit from Takara. The instrument used is the ABI7500 fluorescent quantitative PCR instrument. The real-time fluorescent quantitative PCR primer sequences used in the experiment are as follows:

| | |
|---|---|
| ACTIN-F | 5'-ACACTGTGCCCATCTACGAG-3' (SEQ ID NO.5) |
| ACTIN-R | 5'-CAGCACTGTGTTGGCATAGAG-3' (SEQ ID NO.6) |
| HMGCR-F | 5'-ATCATGTGCTGCTTCGGCTGCAT-3' (SEQ ID NO.7) |
| HMGCR-R | 5'-AAATTGGACGACCCTCACGGCT-3' (SEQ ID NO.8) |
| CYP7A1-F | 5'-TCAAAGAGCGCTGTCTGGGTCA-3' (SEQ ID NO.9) |
| CYP7A1-R | 5'-TTTCCCGGGCTTTATGTGCGGT-3' (SEQ ID NO.10) |
| ABCG1-F | 5'-TGAACCCGTTTCTTTGGCACCG-3' (SEQ ID NO.11) |
| ABCG1-R | 5'-AGTCCCGCATGATGCTGAGGAA-3' (SEQ ID NO.12) |
| FATP1-F | 5'-TGCACAGCAGGTACTACCGCAT-3' (SEQ ID NO.13) |
| FATP1-R | 5'-TGCGCAGTACCACCGTCAAC-3' (SEQ ID NO.14) |
| FABP1-F | 5'-TGGTCCGCAATGAGTTCACCCT-3' (SEQ ID NO.15) |
| FABP1-R | 5'-CCAGCTTGACGACTGCCTTGACTT-3' (SEQ ID NO.16) |
| SREBP1c-F | 5'-GGAGCCATGGATTGCACATT-3' (SEQ ID NO.17) |
| SREBP1c-R | 5'-GCTTCCAGAGAGGAGGCCAG-3' (SEQ ID NO.18) |
| FASN-F | 5'-TCCAAGACTGACTCGGCTACTGAC-3' (SEQ ID NO.19) |
| FASN-R | 5'-GCAGCCAGGTTCGGAATGCTATC-3' (SEQ ID NO.20) |
| PDK4-F | 5'-TTCACACCTTCACCACATGC-3' (SEQ ID NO.21) |
| PDK4-R | 5'-AAAGGGCGGTTTTCTTGATG-3' (SEQ ID NO.22) |
| CPT1-F | 5'-ACCACTGGCCGCATGT-3' (SEQ ID NO.23) |
| CPT1-R | 5'-CTCCATGGCGTAGTAGTTGCT-3' (SEQ ID NO.24) |
| ACOX1-F | 5'-CGGAAGATACATAAAGGAGACC-3' (SEQ ID NO.25) |
| ACOX1-R | 5'-AAGTAGGACACCATACCACCC-3' (SEQ ID NO.26) |
| MCAD-F | 5'-GCTAGTGGAGCACCAAGGAG-3' (SEQ ID NO.27) |
| MCAD-R | 5'-CCAGGCTGCTCTCTGGTAAC-3' (SEQ ID NO.28) |
| UCP2-F | 5'-GCTGGTGGTGGTCGGAGATA-3' (SEQ ID NO.29) |
| UCP2-R | 5'-ACTGGCCCAAGGCAGAGTT-3' (SEQ ID NO.30) |

### 5. Detection of expression of SREBP1/ACC/FASN in liver tissue by Western blot

About 50 mg of liver tissue was taken, 1 ml of Ripa protein lysis buffer (containing a protease inhibitor) was added, and the liver tissue was ground thoroughly and centrifuged at 12000 g and 4°C for 20 min. The supernatant was taken to detect the protein concentration by a BCA method. The protein was quantified to 2 mg/ml with a Ripa tissue protein lysis buffer and a 5X SDS protein loading buffer, and boiled at 98°C for 5 min in a metal bath. 20 µg of protein solution was taken in a 8% polyacrylamide gel for electrophoresis, and transferred to a PVDF membrane. The primary antibodies are FMO2 (NOVUS, NBP1-85952), SREBP1 (Abcam, ab28481), ACC (CST, 3676), and FASN (Abcam, ab99359), and the secondary antibody is goat anti-rabbit antibody (CST, 7074). After washing thoroughly with PBST, exposure was carried out in a dark room according to instructions of super ECL plus.

### 6. Isolation of primary hepatocytes and establishment of in vitro models

(1) Isolation of primary hepatocytes
   (1.1) The required instruments were autoclaved in advance, and DHANKS and 0.03% collagenase IV were prepared. A catheter was soaked with 75% alcohol; a super-clean bench was disinfected by UV irradiation for 30 min; and a medium, DHANKS and collagenase IV were preheated at 37°C.
   (1.2) Sodium pentobarbital (60 mg/kg) was injected intraperitoneally to anesthetize the mice.
   (1.3) The soaked and disinfected catheter was filled with the preheated DHANKS.
   (1.4) The skin was prepared and disinfected with iodine. The abdominal cavity was opened in the super-clean bench to fully expose the portal vein, 1 cm was separated and threaded, and the portal vein was fixed by intubation.
   (1.5) The portal vein was perfused with the DHANKS at a flow rate of 2 mL/min, and the liver became white after perfusion with 20 mL of DHANKS. Then the portal vein was perfused with the preheated collagenase IV at a flow rate of 2 mL/min, and the liver was removed after perfusion with 20 mL of preheated collagenase IV.
   (1.6) The liver was washed with preheated DHANKS, and placed in a mixture of 10 mL of collagenase IV and 10 mL of DHANKS, and the envelope was gently torn off with curved forceps to suspend the hepatocytes in the mixture.
   (1.7) The hepatocyte suspension was digested in a 37°C water bath with shaking for 20 min, and filtered with a 200-mesh screen.
   (1.8) The filtered hepatocyte suspension was centrifuged at 800 rpm for 5 min, and the supernatant was discarded. The hepatocytes were suspended in a serum-free DMEM medium and centrifuged at 800 rpm for 5 min, and the supernatant was discarded.
   (1.9) The obtained hepatocyte pellet was resuspended in a high-sugar DMEM medium containing 10% FBS, and the viability was observed by trypan blue staining. The proportion of living cells was calculated, and the suspended cells were spread in a 6-well plate according to 10⁶ cells per well.
   (1.10) The cells were incubated at 37°C. After 1 h, the medium was changed to 10% FBS high-sugar DMEM. After another 3 h, the medium was changed to a high-sugar DMEM medium containing 2% FBS. The cell morphology was observed after 12 h.
(2) Cell counting method: The diluted cell suspension was dropped on a counting plate to make the suspension freely fill the gap under a cover glass without leaving bubbles. Then the cells are observed under a light microscope and the number of cells in large squares at the four corners was counted. When the cells press a line, only the cells on an upper line and a right line are counted, and then the cell concentration is calculated by the following formula: (total number of cells in the large squares/4) × 10000 × dilution factor = number of cells/mL
(3) Cell modeling: 500 µM of oleic acid was added to the isolated primary hepatocytes after 24 h. Then the primary hepatocytes were cultured for another 24 h and stained with oil red O. Samples were collected to extract RNA, protein and triglycerides.
(4) FMO2 overexpression virus transfection

Negative control lentivirus (FMO2-Vector) and FMO2 overexpression lentivirus (FMO2-Over) were added to the isolated primary hepatocytes after 24 h, with an MOI of 20, and the medium was changed 12 h after transfection. After 48 h, 500 µM of oleic acid was added to make a model.

### 7. Oil red O staining of cells

(1) Preparation of a working solution: A stock solution of saturated oil red O was added to double distilled water according to a ratio of 3:2 (oil red O: double distilled water), mixed well, placed at room temperature for 5 min, and filtered for use.
(2) After the medium was pipetted, the cells were rinsed with PBS 3 times, fixed with 4% paraformaldehyde at room temperature for 10 min, washed with PBS for 5 min, stained with the oil red O working solution for 15 min, washed with PBS for 5 min 3 times, and photographed under an inverted optical microscope within 1 h.

### 8. Detection of triglyceride in primary hepatocytes

The cells were washed with PBS 3 times, and 200 µl of a lipid lysis buffer was added to each well of the six-well plate. The cells were scraped off with a scraper and homogenized by ultrasound, and lysed at room temperature for 10 min. 50 µl of homogenate was taken for BCA protein measurement, and the remaining homogenate was placed in a 70°C water bath for 10 min to inactivate lipase. The homogenate was centrifuged at 2000 rpm for 5 min at room temperature, the supernatant was taken for triglyceride quantification, and finally the quantity was converted to TG/protein in µmol/g.

### 9. Detection of expression of SREBP1/ACC/FASN in primary hepatocytes by Western blot

The cells were washed with PBS 3 times, and 100 µl of a Ripa protein lysis buffer (containing a protease inhibitor) was added to each well of the six-well plate. The cells were scraped off with a scraper, homogenized by ultrasound, ground thoroughly and centrifuged at 12000 g and 4°C for 20 min. The supernatant was taken to detect the protein concentration by a BCA method. The protein was quantified to 1 mg/ml with a Ripa tissue protein lysis buffer and a 5X SDS protein loading buffer, and boiled at 98°C for 5 min in a metal bath. 20 µg of protein solution was taken in a 8% polyacrylamide gel for electrophoresis, and transferred to a PVDF membrane. The primary antibodies are FMO2 (NOVUS, NBP1-85952), SREBP1 (Abcam, ab28481), ACC (CST, 3676), and FASN (Abcam, ab99359), and the secondary antibody is goat anti-rabbit antibody (CST, 7074). After washing thoroughly with PBST, exposure was carried out in a dark room according to instructions of super ECL plus.

### 10. De novo lipid synthesis experiment

Primary hepatocytes were isolated and inoculated in a 6-well plate at a density of 10⁶ cells per well, and cultured overnight in serum-free high-sugar DMEM after adherence. The next day, the medium was changed to a high-sugar medium containing 0.2% fatty acid-free BSA. 2 h later, the medium was changed to a DMEM medium containing 0.5 mM OA, and 0.5uCi U-14C-labeled glucose was added to each well. After culturing for 4 h at 37°C, the lipids were extracted by a Folch method: The cells were washed twice with pre-cooled PBS, and the cells in each well were scraped with 200 µl of PBS containing 0.5% Triton. 150 µl of cells were taken and 500 µl of a solution of chloroform and methanol in a ratio of 2:1 was added to vigorously shake for 30 s, and then 125 µl of double distilled water was added after standing on ice for 1 h. After vigorous shaking, the mixture was centrifuged at 4°C and 1000 RPM for 15 min, the lower organic phase was taken, and the entry of carbon 14 into the lipids was measured using a Tri-Carb 2900 TR liquid scintillation counter. All readings were normalized with protein concentration.

### 11. Data analysis

### 1. Deletion of FMO2 gene aggravates NAFLD induced by high-fat diet

In order to study the role of the FMO2 gene in non-alcoholic liver disease, 8-week-old FMO2 knockout mice FMO2^{-/-} and their littermate wild-type control mice (FMO2^{+/+}) were fed with a normal control diet (NCD) and a high-fat diet (HFD) for 12 weeks. After that, the mice were sacrificed and the accumulation of lipids in the liver of the mice was observed. In this study, FMO2 knockout mice were constructed by homologous recombination. Western Blot results in the liver showed that FMO2 was completely knocked out (FIG. 4E). After 12 weeks of HFD feeding, it was observed that the liver of the mice was significantly swollen and white, and the weight of the liver increased significantly, indicating that the liver had excessive accumulation of lipids and fatty liver was successfully induced (FIG. 1A and FIG. 1C). Compared with the wild-type mice, the livers of the FMO2^{-/-} mice after HFD feeding were generally larger and whiter, and the liver weight increased significantly (FIG. 1A and FIG. 1C). In order to further evaluate the severity of fatty liver, the content of triglyceride in liver tissues was detected and H&E staining was performed on paraffin sections of the liver tissues. The results suggest that the triglyceride content in the liver tissues of the mice after 12 weeks of HFD feeding increased significantly, and H&E staining showed a large number of lipid droplets accumulated in hepatic lobules (FIG. 1B and FIG. 1D). Compared with the wild-type mice, the content of triglyceride in the liver tissues of the FMO2^{-/-} mice significantly increased after HFD feeding, and H&E staining showed that liver steatosis was more serious (FIG. 1B and FIG. 1D), indicating that the deletion of the FMO2 gene aggravates NAFLD induced by the high-fat diet.

### 2. Deletion of FMO2 gene aggravates hepatocyte steatosis induced by oleic acid

In order to study the effect of deletion of the FMO2 gene on hepatocyte steatosis in vitro, primary hepatocytes were isolated from the livers of the wild-type mice and FMO2^{-/-} mice. Western Blot results showed that the isolated FMO2^{-/-} primary hepatocytes lacked FMO2 gene expression (FIG. 5B). Compared with the BSA control group, after 24 h of induction with 500 µM of oleic acid, the triglyceride content in the hepatocytes increased significantly, and oil red O staining showed a large number of lipid droplets accumulated in the hepatocytes (FIG. 2A and FIG. 2B). Compared with the control hepatocytes, the triglyceride content of FMO2^{-/-} hepatocytes significantly increased, and the lipid droplets accumulated more in the hepatocytes. The difference was more obvious after oleic acid induction (FIG. 2A and FIG. 2B), indicating that the deletion of the FMO2 gene will aggravate hepatocyte steatosis induced by oleic acid.

### 3. Overexpression of FMO2 gene improves hepatocyte steatosis induced by oleic acid

From the in vivo mouse high-fat feeding model and the in vitro hepatocyte oleic acid induced model, it can be seen that the deletion of the FMO2 gene can aggravate fatty liver. In order to verify the above conclusion on the negative side, primary hepatocytes were isolated from the livers of wild-type mice and infected with the Ubi-FMO2-3FLAG-CBh-gcGFP-IRES-puromycin lentivirus to overexpress the FMO2 gene. At the same time, the hepatocytes of the control group were infected with the Ubi-MCS-3FLAG-CBhg-cGFP-IRES-puromycin negative control lentivirus. Western Blot results showed that infection with the FMO2 overexpression lentivirus could effectively increase the protein level of FMO2 in hepatocytes (FIG. 5C). According to the statistics of the triglyceride content in hepatocytes and oil red O staining, it can be seen that the infection with the FMO2 negative control virus would not affect the steatosis of hepatocytes, but the steatosis of hepatocytes was significantly improved after overexpression of FMO2, especially after oleic acid induction (FIG. 3A and FIG. 3B), indicating that overexpression of the FMO2 gene can improve hepatocyte steatosis induced by oleic acid.

### 4. FMO2 improves NAFLD by inhibiting de novo lipid synthesis mediated by SREBP1/ACC/FASN pathways

NAFLD is mainly characterized by excessive fat deposition in hepatocytes, inflammation and abnormal liver function, and its pathogenesis involves many factors such as heredity, metabolism, environment, and lifestyle. At present, the exact pathogenesis of NAFLD has not yet been fully unequivocal. As a metabolic center of animals, lipid metabolism in the liver is an extremely complex process, including lipid uptake, lipid de novo synthesis, lipid oxidation, etc. Any abnormality in any link may lead to abnormal lipid deposition in the liver.

In order to explore the specific mechanism of FMO2 gene deletion aggravating NAFLD and find out which link in liver lipid metabolism is regulated by FMO2, the expression of a series of lipid metabolism-related genes in the livers of mice fed with the high-fat diet were tested. PCR results showed that compared with the wild-type mice, there was no significant difference in cholesterol synthesis, lipid uptake, and lipid oxidation in the livers of the FMO2^{-/-} mice, but the expression of genes related to lipid de novo synthesis increased (FIG. 4A-FIG. 4D). In order to verify whether FMO2 affects liver lipid de novo synthesis and further regulates NAFLD, lipid de novo synthesis experiments were conducted by using isotope carbon 14-labeled glucose as a substrate. The results showed that the de novo synthesis ability of FMO2^{-/-} hepatocytes significantly increased compared with the control hepatocytes after oleic acid induction (FIG. 5A). The de novo lipid synthesis (DNL) pathway in the liver plays an important role in the occurrence of fatty liver, and the hub is sterol regulatory element binding protein-1 (SREBP1). SREBP1 is an important transcription factor that regulates fatty acid synthesis in the liver. When synthesized, SREBP1 exists in an inactive form (P-SREBP1) in the endoplasmic reticulum. After being regulated by a specific signal, SREBP1 is transported to the Golgi apparatus and further sheared to release a transcriptionally active fragment (N-SREBP1), and N-SREBP1 enters the nucleus to promote the transcription of a series of lipid synthesis-related genes (ACC/FASN/SCD). Western Blot results of the liver showed that the levels of N-SREBP1 and its downstream proteins ACC and FASN in the liver of FMO2^{-/-} mice after HFD were significantly higher than those of the wild-type mice, suggesting that the activation of DNL pathways in the liver was enhanced (FIG. 4E). The same results were found on in vitro primary hepatocytes, and Western Blot results of hepatocytes showed that the levels of N-SREBP1 and its downstream proteins ACC and FASN in FMO2^{-/-} hepatocytes after oleic acid induction were significantly higher than those of control hepatocytes. Correspondingly, the levels of N-SREBP1 and its downstream proteins ACC and FASN in the hepatocytes of the FMO2 overexpression group after oleic acid induction were significantly lower than those of the control hepatocytes, indicating that FMO2 mainly inhibits the SREBP1/ACC/FASN pathways to reduce de novo synthesis of lipids in the liver, and further improve NAFLD.

FIG. 1 is a graph showing the results of in vivo experiments on C57 mice fed with a normal control diet or a high-fat diet for 12 weeks. A is the statistical result of liver weight of wild-type mice and FMO2^{-/-} mice, *P<0.05 WT Versus FMO2^{-/-}; B is the statistical result of liver triglyceride content of the wild-type mice and FMO2^{-/-} mice, *P<0.05 WT Versus FMO2^{-/-}; C is the comparison result of general appearance of liver of the wild-type mice and FMO2^{-/-} mice; and D is the comparison result of HE staining of liver paraffin sections of the wild-type mice and FMO2^{-/-} mice. WT represents the wild-type mouse group and FMO2^{-/-} represents the FMO2 knockout transgenic mouse group. NCD represents the normal control diet group, and HFD represents the high-fat diet group.

FIG. 2 is the results of in vitro experiments on primary hepatocytes of C57 mice. A is the statistical result of the triglyceride content of wild-type primary hepatocytes and FMO2^{-/-} primary hepatocytes, *P<0.05, **P<0.01 WT Versus FMO2^{-/-}; and B is the result of oil red staining of the wild-type primary hepatocytes and FMO2^{-/-} primary hepatocytes. BSA represents a normal control group, and OA represents a fatty liver in vitro model group.

FIG. 3 is the results of in vitro experiments on primary hepatocytes of C57 mice. A is the statistical result of the triglyceride content of the wild-type primary hepatocyte group, the primary hepatocyte group transfected with the FMO2 negative control virus, and the primary hepatocyte group transfected with the FMO2 overexpression virus, **P<0.01 WT Versus FMO2^{-/-}; and B is the oil red staining results of the wild-type primary hepatocyte group, the primary hepatocyte group transfected with the FMO2 negative control virus, and the primary hepatocyte group transfected with the FMO2 overexpression virus. WT represents the wild-type mouse primary hepatocyte group, FMO2-Vector represents the primary hepatocyte group transfected with the FMO2 negative control virus, and FMO2-Over represents the primary hepatocyte group transfected with the FMO2 overexpression virus. BSA represents a normal control group, and OA represents a fatty liver in vitro model group.

FIG. 4 is the experimental results of the in vivo mechanism study on C57 mice. A is the expression of cholesterol synthesis-related genes in the livers of the wild-type mice and FMO2^{-/-} mice fed with the high-fat diet for 12 weeks; B is the expression of fatty acid uptake-related genes in the livers of the wild-type mice and FMO2^{-/-} mice fed with the high-fat diet for 12 weeks; C is the expression of fatty acid and triglyceride synthesis-related genes in the livers of the wild-type mice and FMO2^{-/-} mice fed with the high-fat diet for 12 weeks; D is the expression of fatty acid and triglyceride oxidative metabolism-related genes in the livers of the wild-type mice and FMO2^{-/-} mice fed with the high-fat diet for 12 weeks; and E is the Western blot results of key proteins of the lipid synthesis pathways in the livers of the wild-type mice and FMO2^{-/-} mice fed with the high-fat diet for 12 weeks. WT represents the wild-type mouse group and FMO2^{-/-} represents the FMO2 knockout transgenic mouse group.

FIG. 5 is a graph showing the experimental results of in vitro mechanism study on mouse primary hepatocytes. A is the results of lipid synthesis experiments, recording the lipid de novo synthesis ability of wild-type primary hepatocytes and FMO2^{-/-} primary hepatocytes; B is the Western blot results of key proteins of the lipid synthesis pathways in the wild-type primary hepatocytes and FMO2^{-/-} primary hepatocytes; and C is the Western blot results of key proteins of the lipid synthesis pathway in the FMO2 negative control virus-transfected primary hepatocyte group and the FMO2 overexpression virus-transfected primary hepatocyte group.

The raw materials and equipment used in the present invention, unless otherwise specified, are all commonly used raw materials and equipment in the field; and the methods used in the present invention, unless otherwise specified, are all conventional methods in the field.

## Claims

1. A flavin-containing monooxygenase 2 (FMO2) for use in the treatment of non-alcoholic fatty liver disease (NAFLD).

2. The flavin-containing monooxygenase 2 (FMO2) for use according to claim 1, wherein the flavin-containing monooxygenase 2 (FMO2) has a nucleotide sequence as shown in SEQ ID NO. 1 or SEQ ID NO. 3, and an amino acid sequence as shown in SEQ ID NO. 2 or SEQ ID NO. 4.

3. The flavin-containing monooxygenase 2 (FMO2) for use according to any one of claims 1 or 2, wherein the flavin-containing monooxygenase 2 (FMO2) is administered as an oral preparation or an injection preparation.

4. The flavin-containing monooxygenase 2 (FMO2) for use according to claim 3, wherein the dosage form of the oral preparation is a capsule, a tablet, a solution or powder; and the injection preparation is an injection or powder.

5. A composition comprising a flavin-containing monooxygenase 2 (FMO2) for use in the treatment of non-alcoholic fatty liver disease (NAFLD), wherein said composition further comprises a pharmaceutically acceptable carrier, excipient or solvent.

## Patentansprüche

1. Flavinhaltiger Monooxygenase 2 (FMO2) zur Verwendung bei der Behandlung von nichtalkoholischer Fettlebererkrankung (NAFLD).

2. Flavinhaltiger Monooxygenase 2 (FMO2) zur Verwendung nach Anspruch 1, wobei der flavinhaltigen Monooxygenase 2 (FMO2) eine Nukleotidsequenz, wie in SEQ ID NO. 1 oder SEQ ID NO: 3, und eine Aminosäuresequenz, wie in SEQ ID NO. 2 oder SEQ ID NO: 4, umfasst.

3. Der flavinhaltigen Monooxygenase 2 (FMO2) zur Verwendung nach einem der Ansprüche 1 oder 2, wobei der flavinhaltigen Monooxygenase 2 (FMO2) als ein orales Präparat oder ein Injektionspräparat verabreicht wird.

4. Flavinhaltiger Monooxygenase 2 (FMO2) zur Verwendung nach Anspruch 3, wobei die Dosierungsform der oralen Zubereitung eine Kapsel, eine Tablette, eine Lösung oder ein Pulver ist; und die Injektionszubereitung eine Injektion oder ein Pulver ist.

5. Zusammensetzung, umfassend ein flavinhaltiger Monooxygenase 2 (FMO2) zur Verwendung bei der Behandlung einer nicht-alkoholischen Fettlebererkrankung (NAFLD), wobei die Zusammensetzung ferner einen pharmazeutisch verträglichen Träger, einen Hilfsstoff oder ein Lösungsmittel umfasst.

## Revendications

1. L'invention concerne une monooxygénase 2 (FMO2) contenant de la flavine, destinée à être utilisée dans le traitement de la stéatose hépatique non alcoolique (NAFLD).

2. Monooxygénase 2 contenant de la flavine (FMO2) destinée à être utilisée selon la revendication 1, dans laquelle la monooxygénase 2 contenant de la flavine (FMO2) a une séquence nucléotidique telle que représentée dans NO ID SÉQ. 1 ou NO ID SÉQ. 3, et une séquence d'acides aminés telle que représentée dans NO ID SÉQ. 2 ou NO ID SÉQ. 4.

3. Monooxygénase 2 contenant une flavine (FMO2) destinée à être utilisée selon l'une quelconque des revendications 1 ou 2, dans laquelle la monooxygénase 2 contenant une flavine (FMO2) est administrée sous forme d'une préparation orale ou d'une préparation injectable.

4. Monooxygénase 2 contenant de la flavine (FMO2) à utiliser selon la revendication 3, dans laquelle la forme posologique de la préparation orale est une capsule, un comprimé, une solution ou une poudre ; et la préparation d'injection est une injection ou une poudre.

5. Composition comprenant une monooxygénase 2 contenant de la flavine (FM02), destinée à être utilisée dans le traitement de la stéatose hépatique non alcoolique (NAFLD), dans laquelle ladite composition comprend en outre un support, un excipient ou un solvant pharmaceutiquement acceptable.
